# EUROPEAN PATENT APPLICATION

(11) **EP 4 207 229 A1**
(43) Date of publication of application: **05.07.2023**
(21) Application number: 21859908.2
(22) Date of filing: 08.07.2021
(51) Int. Cl.: H01H 13/04, H01H 13/14, H01H 13/28

(54) **ELECTRONIC DEVICE AND WEARABLE ELECTRONIC DEVICE**

(30) Priority: 31.08.2020 CN 202010902435; 31.08.2020 CN 202021870422 U
(71) Applicant: GUANGDONG OPPO MOBILE TELECOMMUNICATIONS CORP., LTD., Dongguan, Guangdong 523860 (CN)
(72) Inventor: WEI, Yongzhang, Dongguan, Guangdong 523860 (CN); GENG, Jingzhuo, Dongguan, Guangdong 523860 (CN); ZHANG, Xintao, Dongguan, Guangdong 523860 (CN)
(74) Representative: Taor, Simon Edward William
(86) International application number: PCT/CN2021/105171
(87) International publication number: WO 2022/042068

(57) **Abstract**

An electronic device and a wearable electronic device are provided in implementations of the disclosure. The electronic device (100) includes a housing frame (1), a button assembly (4), and a circuit assembly (5). The housing frame (1) has an inner cavity (102) and a first through hole (103). The first through hole (103) communicates with the inner cavity (102) and an external space (400) outside the housing frame (1). The button assembly (4) includes a first conductive member (41) and a second conductive member (42). The first conductive member (41) is connected to the housing frame (1) and at least partially in the external space (400). The second conductive member (42) is at least partially in the first through hole (103). The first conductive member (41) elastically abuts against the second conductive member (42), so that the first conductive member (41) is in electrical communication with the second conductive member (42). The circuit assembly (5) is disposed in the inner cavity (102). The circuit assembly (5) includes a circuit board (51) and an electrical contact portion (52) electrically connected to the circuit board (51). The electrical contact portion (52) abuts against an end of the second conductive member (42) away from the first conductive member (41) so that the first conductive member (41) is in electrical communication with the electrical contact portion (52). The electronic device and the wearable electronic device each are provided with the button assembly having good impact resistance.

## Description

### TECHNICAL FIELD

This disclosure relates to the field of electronic technology, and in particular, to an electronic device and a wearable electronic device.

### BACKGROUND

With increasing pursuit of functions of electronic devices, users have increasing requirements for button assemblies of electronic devices. The button assembly may be used as an electrode for detecting biometric features. During use of the electronic device, the button assembly is easily impacted in collision or drop due to being located on an outer surface of the electronic device, resulting in adverse effects on a circuit assembly connected to the button assembly. To solve above technical problems, in the disclosure, an electronic device is provided with a button assembly having good impact resistance.

### SUMMARY

In the disclosure, an electronic device and a wearable electronic device which each are provided with a button assembly having good impact resistance.

In a first aspect, an electronic device is provided in implementations of the disclosure. The electronic device includes a housing frame, a button assembly, and a circuit assembly. The housing frame has an inner cavity and a first through hole, the first through hole communicates with the inner cavity and an external space outside the housing frame. The button assembly includes a first conductive member and a second conductive member, the first conductive member is connected to the housing frame and at least partially in the external space, the second conductive member is at least partially in the first through hole, and the first conductive member elastically abuts against the second conductive member, so that the first conductive member is in electrical communication with the second conductive member. The circuit assembly is disposed in the inner cavity, the circuit assembly includes a circuit board and an electrical contact portion electrically connected to the circuit board, and the electrical contact portion abuts against an end of the second conductive member away from the first conductive member so that the first conductive member is in electrical communication with the electrical contact portion.

In a second aspect, a wearable electronic device is provided in the implementations of the disclosure. The wearable electronic device includes a wearable member and the above-mentioned electronic device. The wearable member is connected to the electronic device, and the electronic device comprises at least one detection electrode, and the at least one detection electrode contacts a detection site of an object to-be-detected when the wearable electronic device is worn on the object to-be-detected.

### BRIEF DESCRIPTION OF THE DRAWINGS

To describe the technical solutions in the implementations of the disclosure more clearly, the following briefly introduces the accompanying drawings required for describing the implementations. Apparently, the accompanying drawings in the following description illustrate some implementations of the disclosure. Those of ordinary skill in the art may also obtain other drawings based on these accompanying drawings without creative efforts.
FIG. 1 is a schematic structural view of a wearable electronic device provided in implementations of the disclosure.
FIG. 2 is a perspective view of an electronic device provided in implementations of the disclosure.
FIG. 3 is a bottom view of an electronic device provided in implementations of the disclosure.
FIG. 4 is a schematic exploded view of an electronic device provided in implementations of the disclosure.
FIG. 5 is schematic partial structural view of an electronic device provided in a first implementation of the disclosure.
FIG. 6 is a schematic enlarged view illustrating a button assembly illustrated in FIG. 5.
FIG. 7 is a schematic exploded view illustrating a housing frame, a button assembly, and a circuit assembly provided in FIG. 5.
FIG. 8 is an exploded cross-sectional view illustrating the housing frame, the button assembly, and the circuit assembly illustrated in FIG. 7.
FIG. 9 is a schematic enlarged view illustrating a button assembly provided in another implementation of the disclosure.
FIG. 10 is a schematic enlarged view illustrating a button assembly provided in a second implementation of the disclosure.
FIG. 11 is a schematic exploded view illustrating a housing frame, the button assembly, and a circuit assembly provided in the second implementation of the disclosure.
FIG. 12 is an exploded cross-sectional view illustrating the housing frame, the button assembly, and the circuit assembly illustrated in FIG. 11.
FIG. 13 is a schematic enlarged view illustrating a button assembly provided in a third implementation of the disclosure.
FIG. 14 is a schematic enlarged view illustrating a button assembly provided in a fourth implementation of the disclosure.
FIG. 15 is a schematic structural view illustrating a button assembly provided in a fifth implementation of the disclosure.
FIG. 16 is a schematic structural view of an elastic conductive member provided in implementations of the disclosure.
FIG. 17 is a schematic structural view of a button assembly provided in a sixth implementation of the disclosure.
FIG. 18 is a schematic structural view of another elastic conductive member provided in implementations of the disclosure.

### DETAILED DESCRIPTION

The technical solutions in the implementations of the disclosure are clearly and completely described in the following with reference to the accompanying drawings in the implementations of the disclosure. Apparently, the described implementations are merely part rather than all of the implementations of the disclosure. The implementations listed in the disclosure may be properly combined with each other.

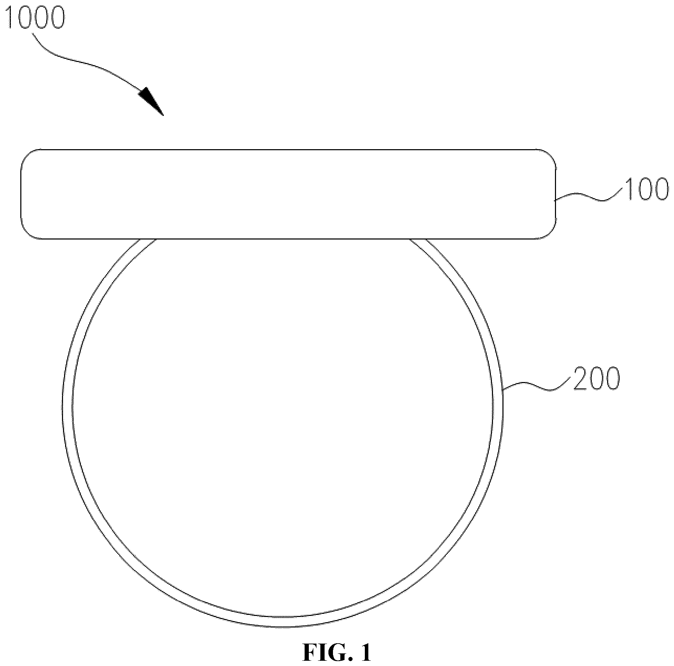

Refer to FIG. 1, an electronic device 100 provided in the disclosure may be applied to a wearable electronic device 1000. The wearable electronic device 1000 may include but is not limited to electronic devices such as a smart watch, a smart bracelet, smart glasses, a smart helmet, a smart earphone, a smart necklace, and a smart ring. Optionally, the wearable electronic device 1000 is provided with a sensor therein, and a detection electrode connected to the sensor may be provided on a housing of the wearable electronic device 1000. The wearable electronic device 1000 may be worn on an object (for example, human body, animal, article, etc.), and the detection electrode can detect feature information of the object when the detection electrode contacts the object. In the disclosure, a smart watch is taken as an example for illustrating the wearable electronic device 1000.

The sensor configured to detect feature information may include but is not limited to a heart rate sensor, a biosensor, an electrodermal sensors, and the like.

The heart rate sensor may be a photoelectric heart rate sensor that takes measurements based on light reflection, or an electrode-type heart rate sensor that takes measurements based on potentials of different parts of the human body.

The Biosensor may include but is not limited to a blood glucose sensor for detecting blood sugar, a blood pressure sensor for detecting blood pressure, an electrocardiographic sensor for detecting electrocardiograms, a myoelectric sensor for detecting myoelectricity, a body temperature sensor for detecting body temperature, a brain wave sensor for detecting brain waves, etc.

The electrodermal sensor for detecting skin resistance or conductance may be used for lie detection or detecting a time period of high learning efficiency or working efficiency.

The wearable electronic device 1000 may also be provided with a motion sensor, an environment sensor, etc., so that the wearable electronic device 1000 may integrate more functions. The motion sensor may include but is not limited to an acceleration sensor, a gyroscope, a geomagnetic sensor (e.g., an electronic compass sensor), an atmospheric pressure sensor, and the like. The environmental sensor may include but is not limited to an air temperature and humidity sensor, a rain sensor, a light sensor, a wind speed and direction sensor, and the like.

Refer to FIG. 1, the wearable electronic device 1000 is provided in the implementations of the disclosure. The wearable electronic device 1000 includes the electronic device 100 and a wearable member 200. The wearable member 200 is connected to the electronic device 100. The electronic device 100 is a functional body of the wearable electronic device 1000. The wearable member 200 may be a connection structure such as a bracelet, a ring, a spectacle frame, and the like, and by means of the wearable member 200, the electronic device can be worn on the object.

The electronic device 100 may be fixedly connected to the wearable member 200, or the electronic device 100 may be detachably connected to the wearable member 200.

In the implementations, the electronic device 100 that can detect electrocardiogram is taken as an example for illustrating structures of the electronic device 100 in detail. For structure design of the electronic device 100 that can detect other feature information, reference can be made to the implementation, which also falls into the scope of the disclosure.

The electronic device 100 may include an electrocardiogram detector. The electrocardiogram detector may include one or more detection electrodes and a signal acquisition circuit connected to the detection electrodes.

The detection electrode is made of a conductive material. The detection electrode may be made of a material that includes but is not limited to metal, metal oxide, graphene, conductive graphite, conductive carbon black, single-walled and multi-walled carbon nanotubes. The metal in the detection electrode may be in a form of metal nanowires, metal nanoparticles, or metal oxide nanoparticles. The composition of the metal includes but is not limited to gold, silver, copper, aluminum, or nickel. The composition of metal oxide includes but is not limited to indium tin oxide (ITO) or fluorine doped tin oxide (FTO).

The electronic device 100 for electrocardiogram detection in the implementations may include at least three detection electrodes.

The detection electrodes may be disposed on an outer surface of the housing of the electronic device 100. When the wearable electronic device 1000 is worn on an object to-be-detected (that is, the above-mentioned object), the detection electrodes contact detection sites of the object to-be-detected to detect the electrocardiogram of the object to-be-detected. A smart watch is taken as example for illustrating the wearable electronic device 1000. The electronic device 100 may be a dial of the smart watch, and the wearable member 200 may be a strap of the smart watch. The dial may be circular, square, curved, special-shaped, etc. In the implementations, a square dial is taken as example for illustration.

For ease of illustration, a length direction of the electronic device 100 is defined as Y-axis direction, a width direction of the electronic device 100 is defined as X-axis direction, and a thickness direction of the electronic device 100 is defined as Z-axis direction.

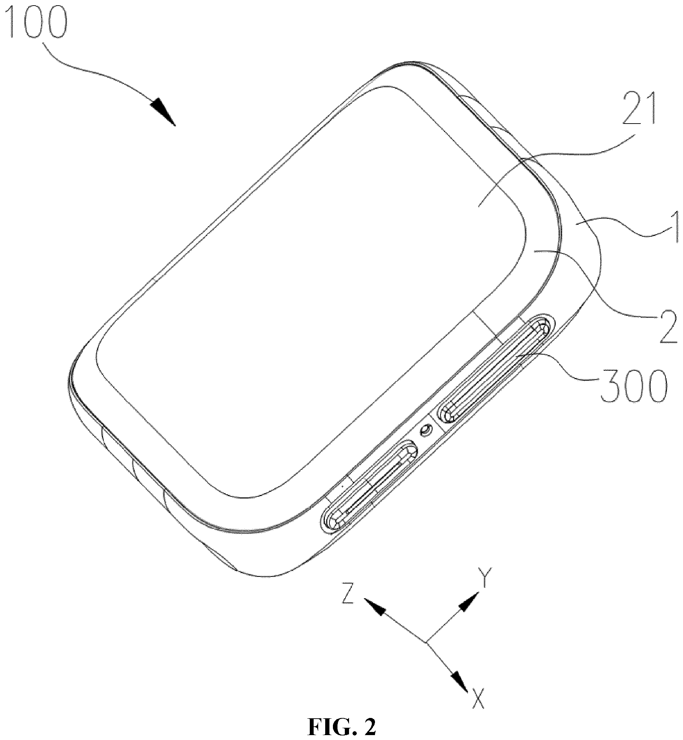

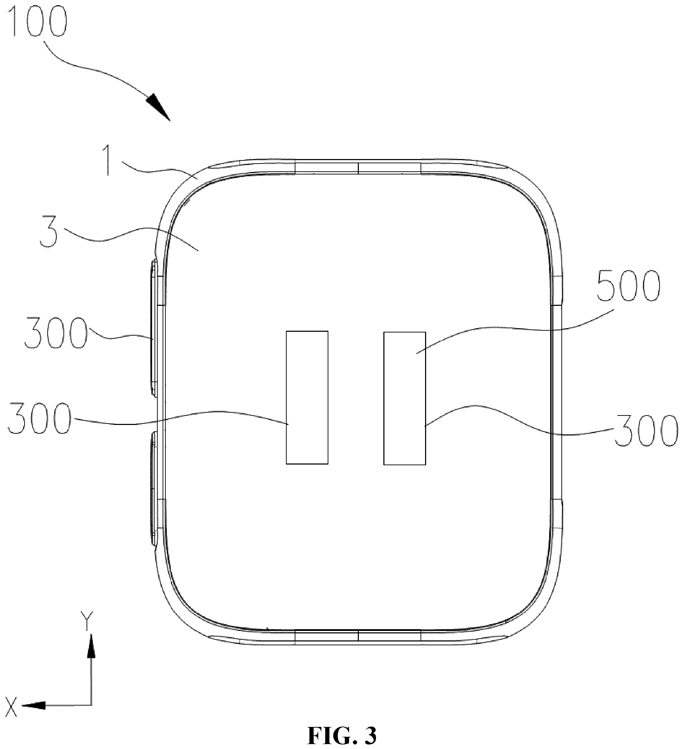

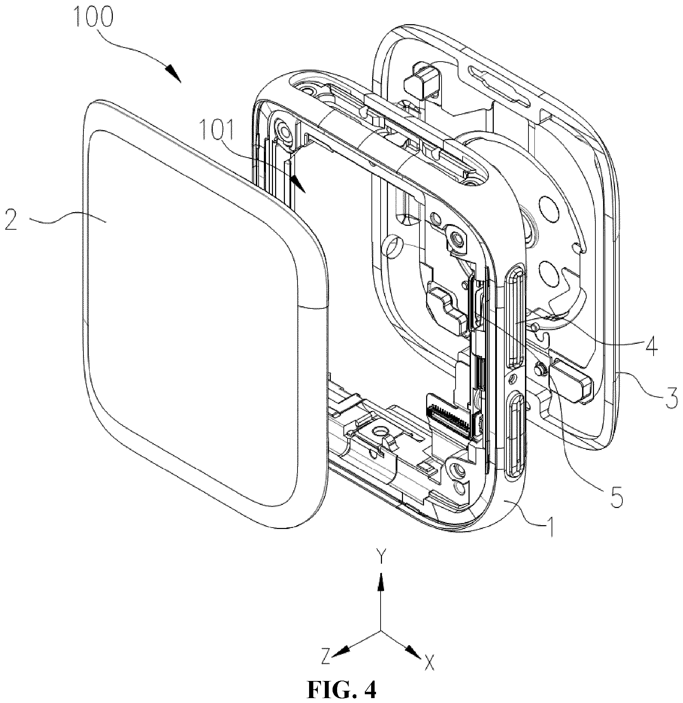

Refer to FIG. 2, the electronic device 100 includes a housing frame 1. Optionally, the housing frame 1 may be a side frame of electronic device 100. Specifically, refer to FIG. 3 and FIG. 4, the electronic device 100 further includes a top housing 2 and a bottom housing 3 opposite the top housing 2, and the housing frame 1 is connected between the top housing 2 and the bottom housing 3. The top housing 2, the housing frame 1, and the bottom housing 3 cooperatively define a sealed or substantially sealed receiving cavity 101. The receiving cavity 101 may accommodate components such as circuit boards, sensors, batteries, and the like. In other words, the top housing 2, the housing frame 1, and the bottom housing 3 cooperatively form the entire housing of the electronic device 100.

Refer to FIG. 3, when the wearable electronic device 1000 is worn on the object to-be-detected, the bottom housing 3 of the electronic device 100 may contact the wrist of the object to-be-detected. In the implementations, there are three detection electrodes 300. The three detection electrodes 300 are merely taken as an example, and the number of detection electrodes 300 is not limited thereto. Optionally, two detection electrodes 300 may be disposed on an outer surface of the bottom housing 3, and one detection electrode 300 may be disposed an outer surface of the housing frame 1, which will be used for illustrating the implementations hereinafter. In other optional implementations, two detection electrodes 300 may be disposed on the bottom housing 3, and another one detection electrode 300 may be disposed on the top housing 2. The two detection electrodes 300 disposed on the bottom housing 3 are independent of each other, that is, the two detection electrodes 300 are not in direct contact with each other. When the wearable electronic device 1000 is worn on the object to-be-detected, the two detection electrodes 300 on the bottom housing 3 are always in contact with the object to-be-detected. The two detection electrodes 300 on the bottom housing 3 extend from the outer surface of the bottom housing 3 to the receiving cavity 101 and are connected to the signal acquisition circuit in the receiving cavity 101. Another detection electrode 300 is also connected to the signal acquisition circuit in the receiving cavity 101.

When the wearable electronic device 1000 is worn on the left hand of the object to-be-detected, the two detection electrodes 300 are in contact with the left hand of the object to-be-detected. The left hand is merely taken as an example, and a position where the wearable electronic device 1000 is worn is not limited thereto. Here, a conductive loop is the left hand - the detection electrodes 300 on the bottom housing 3 - the signal acquisition circuit. When the detection electrode 300 on the housing frame 1 is touched or pressed by the right hand of the object to-be-detected, a conductive loop is the right hand - the detection electrode 300 on the housing frame 1 - the signal acquisition circuit. The signal acquisition circuit is configured to collect a voltage difference between the left hand and the right hand, and then form an electrocardiogram waveform according to the voltage difference. One of the two detection electrodes 300 on the bottom housing 3 may be a driving electrode, which is used to enhance an anti-interference ability of the signal acquisition circuit.

Optionally, the housing frame 1 in the disclosure may also form the entire housing of the electronic device 100. In other words, the button assembly 4 of the electronic device 100 in the disclosure may be located at the top housing 2, the bottom housing 3, or a side housing.

Optionally, the bottom housing 3 and the side housing of the electronic device 100 may integrally form the housing frame 1. In other words, the button assembly 4 of the electronic device 100 in the disclosure may be located at the bottom housing 3 or the side housing.

The specific structure of the electronic device 100 will be illustrated below in conjunction with the accompanying drawings.

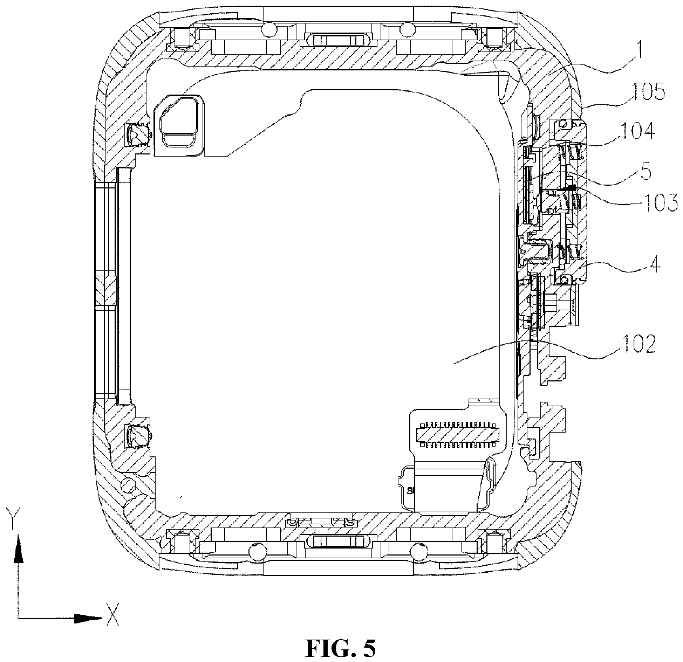

Refer to FIG. 4 and FIG. 5, the electronic device 100 includes the housing frame 1, the button assembly 4, and a circuit assembly 5.

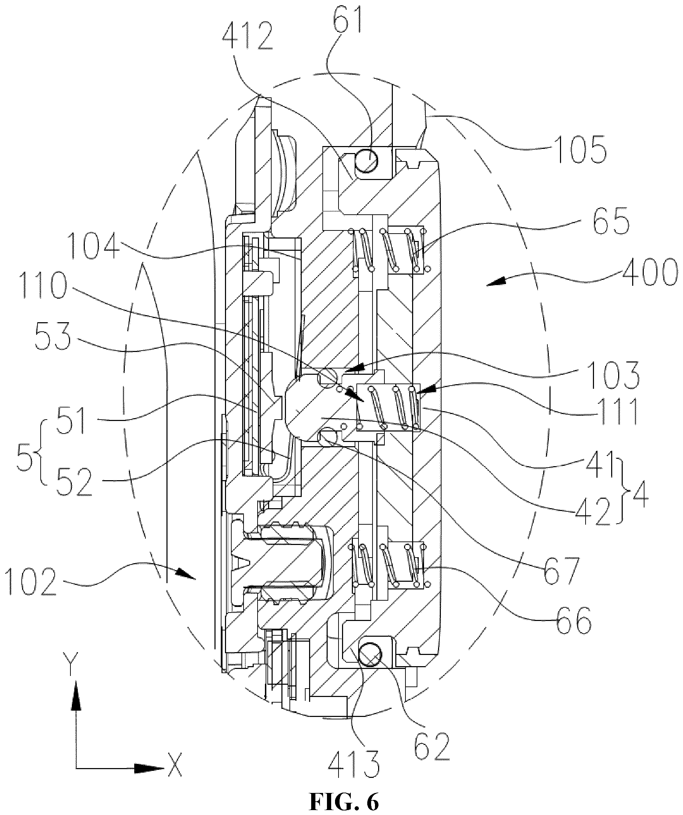

In the implementations, refer to FIG. 5 and FIG. 6, the housing frame 1 forms the side housing. The housing frame 1 defines an inner cavity 102 and a first through hole 103. The first through hole 103 communicates with the inner cavity 102 and an external space 400 outside the housing frame 1. In other words, the housing frame 1 includes an inner surface 104 in an annular shape and an outer surface 105 in an annular shape, and the inner surface 104 is opposite to the outer surface 105. A space defined by the inner surface 104 is the inner cavity 102 of the housing frame 1. In a case that the electronic device 100 has the top housing 2 and the bottom housing 3, a surface of the top housing 2 facing the bottom housing 3, the annular inner surface 104 of the housing frame 1, and a surface of the bottom housing 3 facing the top housing 2 cooperatively define the inner cavity 102. The external space 400 may be a space at a side of the annular outer surface 105 of the housing frame 1 away from the annular inner surface 104. Optionally, the inner surface 104 of the housing frame 1 may have flat surfaces, curved surfaces, uneven surfaces, and the like. The outer surface 105 of the housing frame 1 may have flat surfaces, curved surfaces, uneven surfaces, and the like. The housing frame 1 may be an independent structure made of one material, or a composite structure or a spliced structure made of various different materials through lamination, injection molding, and other processes.

Refer to FIG. 6, the first through hole 103 extends from the inner surface 104 of the housing frame 1 to the outer surface 105 of the housing frame 1. The structure of the first through hole 103 is not limited herein. For example, the first through hole 103 may be a circular hole, a square hole, an oval hole, a rhombus hole, a pentagonal hole, a hexagonal hole, or other special-shaped holes.

Refer to FIG. 6, the button assembly 4 includes a first conductive member 41 and a second conductive member 42. The first conductive member 41 and the second conductive member 42 may be regarded as part of the detection electrode 300 disposed on the housing frame 1 or may be regarded as the entire detection electrode 300 disposed on the housing frame 1. The first conductive member 41 and the second conductive member 42 may both be made of a conductive material(s). A material(s) of the first conductive member 41 and the second conductive member 42 may refer to above-mentioned material of the detection electrode 300.

Refer to FIG. 6, the first conductive member 41 may be in direct connection with or indirect connection with the housing frame 1. The direct connection refers to that two components contact with each other. The indirect connection refers to that two components do not contact with each other and are connected via other components. The first conductive member 41 may be at least partially in the external space 400, for example, a surface or some three-dimensional portions of the first conductive member 41 is in the external space 40, so that the first conductive member 41 can be directly contacted by the object to-be-detected. The entire first conductive member 41 may also be disposed in the external space 400. The second conductive member 42 may be at least partially in the first through hole 103. Optionally, part of the first conductive member 41 is in the first through hole 103. Optionally, the entire first conductive member 41 is disposed in first through hole 103.

Refer to FIG. 6, the first conductive member 41 elastically abuts against the second conductive member 42. The first conductive member 41 may directly abut against the second conductive member 42. The first conductive member 41 may also indirectly abut against the second conductive member 42, that is, the first conductive member 41 may abut against the second conductive member 42 via other structures. Term "abutting" indicates that two abutments are connected, and there is a force greater than or equal to zero between the two abutments in a connection direction.

The first conductive member 41 and the second conductive member 42 may be in electrical communication. In other words, a conductive branch may be formed between the first conductive member 41 and the second conductive member 42.

Refer to FIG. 6, the circuit assembly 5 is disposed in the inner cavity 102. The circuit assembly 5 includes a circuit board 51, an electrical contact portion 52 disposed on the circuit board 51, and a signal acquisition circuit (not illustrated). The circuit board 51 may be a flexible circuit board. The electrical contact portion 52 is connected to the signal acquisition circuit. Specifically, the electrical contact portion 52 and the signal acquisition circuit may be disposed on different circuit boards. For example, the electrical contact portion 52 may be disposed on a flexible circuit board, the signal acquisition circuit may be disposed on a main board, and the flexible circuit board is connected to the main board. In other optional implementations, the electrical contact portion 52 and the signal acquisition circuit may be disposed on the same circuit board. For example, the electrical contact portion 52 and the signal acquisition circuit are both disposed on the flexible circuit board; or the electrical contact portion 52 and the signal acquisition circuit are both disposed on the main board.

Refer to FIG. 6, the electrical contact portion 52 abuts against an end of the second conductive member 42 away from the first conductive member 41, so that the first conductive member 41 can be in electrical communication with the electrical contact portion 52. When the first conductive member 41 is in direct contact with the object to-be-detected, the object to-be-detected, the first conductive member 41, the second conductive member 42, the electrical contact portion 52, and the signal acquisition circuit are in electrical communication, and thus the signal acquisition circuit can collect electrocardiogram information of the object to-be-detected. The first conductive member 41 may be part of the detection electrode 300 for detecting biological information or serve as the entire detection electrode 300 for detecting biological information. Optionally, the first conductive member 41, the second conductive member 42, and the electrical contact portion 52 may sequentially abut in the X-axis direction, the Y-axis direction, or the Z-axis direction. In the implementations, as illustrated in FIG. 6, the first conductive member 41, the second conductive member 42, and the electrical contact portion 52 sequentially abut in the X-axis direction.

In the implementations, refer to FIG. 3, the bottom housing 3 is also provided with a third conductive member 500. The third conductive member 500 is at least partially located on the outer surface 105 of the bottom housing 3. The third conductive member 500 is connected to the signal acquisition circuit. The third conductive member 500 and the first conductive member 41 together form the detection electrode 300 for detecting biological information. The third conductive member 500 corresponds to one or both of the two detection electrodes 300 on the bottom housing 3. The third conductive member 500 is located on the outer surface 105 of bottom housing 3. When the wearable electronic device 1000 is worn on the object to-be-detected, the signal acquisition circuit, the second conductive member 42, the first conductive member 41, the object to-be-detected, and third conductive member 500 form a conductive loop to detect biological information of the object to-be-detected. The biological information is the information collected by the signal acquisition circuit. In the implementations, the biological information is electrocardiogram information.

The third conductive member 500 may omitted by those skilled in the art according to actual needs. For example, a complete technical solution for detecting feature information such as temperature, pulse, and blood oxygen may be formed in the implementations without the third conductive member 500.

For the position of the first conductive member 41, the position of the second conductive member 42, and the position of the third conductive member 500, according to actual needs, those skilled in the art may make reasonable changes without creative efforts, which all fall into the scope of the disclosure.

A one-piece button assembly may directly transmit a received impact force to the electrical contact portion. As a result, on the one hand, the button assembly itself may be broken due to a large impact force, and on the other hand, the electrical contact portion may also be broken due to the large impact force, resulting in a damage to a structure of the electronic device and a poor impact resistance of the electronic device.

In the electronic device 100 provided in the implementations of the disclosure, a splittype button assembly 4 is provided. The button assembly 4 may serve as one detection electrode 300. The button assembly 4 is provided with two portions elastically connected with each other, and when the first conductive member 41 is collided, an impact force can be at least partially absorbed by an elastic abutment between the first conductive member 41 and the second conductive member 42, so that a receding distance of the second conductive member 42 caused by the impact force can be reduced or the second conductive member 42 may even not recede, thereby reducing adverse effects on the button assembly 4 itself or on the circuit assembly 5 applied by the button assembly 4.

In the implementations, refer to FIG. 2, the top housing 2 includes a display screen 21. The display screen 21 is configured to display the biological information detected by the signal acquisition circuit, so that a detection result can be visually observed by the object to-be-detected.

Optionally, the electrical contact portion 52 is disposed on the circuit board 51, and the circuit board 51 faces the inner surface 104 of the housing frame 1. The circuit assembly 5 further includes a functional circuit (not illustrated) and a switch unit 53. The functional circuit is connected to the switch unit 53. The functional circuit may be disposed on the circuit board 51 or the main board. The switch unit 53 may be a button switch. When the switch unit 53 is not pressed, the functional circuit is in a short circuit state. When the switch unit 53 is pressed, the functional circuit is in a conducting state. The functional circuit is electrically connected to a control chip of the main board. When in the conducting state, the functional circuit may be used to realize one or more of functions such as turning on the display screen 21, turning off the display screen 21, increasing the volume, decreasing the volume, taking pictures, enabling an application program, disabling an application program, turning on a camera, turn offing a camera, etc.

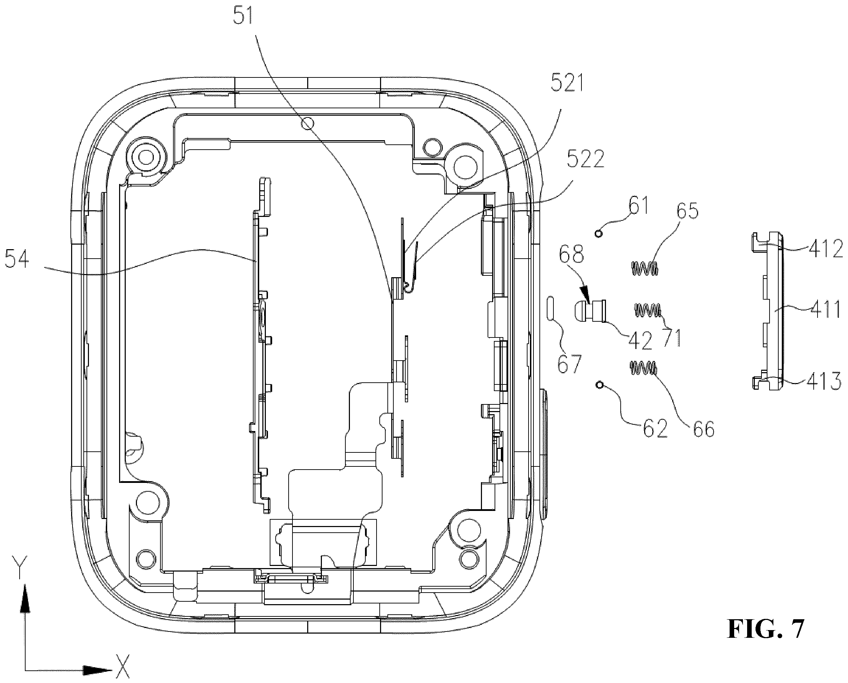

Refer to FIG. 7, the circuit board 51 may be a flexible circuit board. The electronic device 100 further includes a circuit board support 54 for supporting the circuit board 51. The switch unit 53 is disposed on the circuit board 51. Further, the electrical contact portion 52 may be a conductive resilient piece. The electrical contact portion 52 is on the circuit board 51, the second conductive member 42 and the electrical contact portion 52 are in an interference fit with (that is, abut against) each other. The first conductive member 41 is elastically connected to the second conductive member 42. In this way, it is possible to ensure a good electrical communication and low impedance between the first conductive member 41 and the electrical contact portion 52, and normal detection functions such as electrocardiogram detection. The electrical contact portion 52, the first conductive member 41, and the second conductive member 42 may be partially or completely gold-plated to improve electrical conductivity and stability.

Refer to FIG. 6 and FIG. 7, the electrical contact portion 52 includes a fixed end 521 and an abutting end 522 connected to the fixed end 521. The fixed end 521 is fixed on the circuit board 51. The fixed end 521 is connected to the signal acquisition circuit. The abutting end 522 is disposed at a side of the switch unit 53 away from the flexible circuit board 51. The abutting end 522 is opposite to and spaced apart from the switch unit 53. The abutting end 522 abuts against the end of the second conductive member 42 away from the first conductive member 41.

When the first conductive member 41 is pressed to push the second conductive member 42 to move, the second conductive member 42 pushes the abutting end 522 to press the switch unit 53, so that the functional circuit can be triggered to generate a trigger signal to realize functions of the functional circuit.

In other words, the button assembly 4 provided in the implementations of the disclosure may not only serve as the detection electrode 300 of the signal acquisition circuit, but also serve as a trigger button of the functional circuit, so that a multi-purpose of the button assembly 4 is realized, and functions of the detection electrode 300 and the trigger button are combined, thereby realizing function integration, reducing the number of structures, and saving cost and space.

Optionally, the first conductive member 41 may serve as a button cap of the button assembly 4, and the second conductive member 42 may serve as a button post of the button assembly 4. The button cap may be touched or pressed by the hand or other parts of the object to-be-detected, and the button post is configured to transmit a current signal, a pressing force, etc. to the electrical contact portion 52.

Structures of the housing frame 1, the first conductive member 41, and the second conductive member 42 are exemplified hereinafter in conjunction with the accompanying drawings. The structures of the housing frame 1, the first conductive member 41, and the second conductive member 42 provided in the disclosure include but are not limited to structures in the following implementations.

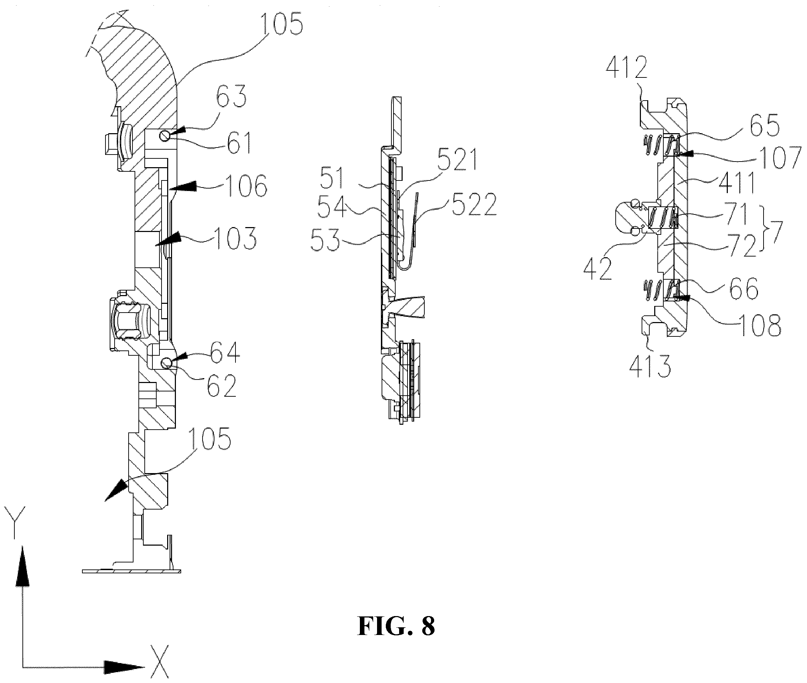

Optionally, refer to FIG. 8, the housing frame 1 defines a receiving groove 106 on an outer peripheral wall (i.e., the outer surface 105) of the housing frame 1. The first through hole 103 communicates with the receiving groove 106 and the inner cavity 102 of the housing frame 1. In other words, the receiving groove 106 has an opening on the outer surface 105 of the housing frame 1. A bottom surface of the receiving groove 106 is opposite to the opening of the receiving groove 106. The first through hole 103 extends through the bottom surface of the receiving groove 106.

The first conductive member 41 is at least partially disposed in the receiving groove 106. Optionally, a part of the first conductive member 41 may be in the receiving groove 106. Another part of the first conductive member 41 may exceed the outer surface 105 of the housing frame 1. In other words, an outer surface 105 of the first conductive member 41 may slightly exceed the outer surface 105 of the housing frame 1 to form a protrusion portion that is pressable. Optionally, yet another part of the first conductive member 41 may also be in the first through hole 103.

Optionally, the housing frame 1 may be made of metal, so that the housing of the electronic device 100 has a relatively high strength. At least part of the button assembly 4 is made of a conductive material. The button assembly 4 is insulated from the housing frame 1. Optionally, the housing 1 is provided with an insulating sleeve, an insulating film, an insulating layer, or the like on a surface of the button assembly 4 facing metal surfaces of the housing frame 1. The insulating film is taken as an example. The insulating film is used to insulate the button assembly 4 from the metal surfaces of the housing frame 1. Optionally, the insulating film may also be disposed on multiple metal surfaces of the housing frame 1 facing the button assembly 4.

Optionally, the housing frame 1 may be partially made of a non-conductive material or the entire housing frame 1 may be made of a non-conductive material. Part of the housing frame 1 that accommodates the button assembly 4 may be made of a non-conductive material, so that there is no conduction between the housing frame 1 and the button assembly 4.

Optionally, in at least one of the X-axis direction or the Y-axis direction, a size of the receiving groove 106 may be greater than that of the first through hole 103. In other optional implementations, in at least one of the X-axis direction or the Y-axis direction, the size of the receiving groove 106 may be equal to that of the first through hole 103. Alternatively, in at least one of the X-axis direction or the Y-axis direction, the size of the receiving groove 106 may be less than that of the first through hole 103.

In other implementations, the housing frame 1 may not define the receiving groove 106 on the outer peripheral wall of the housing frame 1, a part of the first conductive member 41 is in the first through hole 103, and another part of the first conductive member 41 exceeds the outer surface 105 of the housing frame 1.

The first conductive member 41 may be mounted in the receiving groove 106 in a manner that includes but is not limited to the following implementations.

Optionally, refer to FIG. 7 and FIG. 8, the electronic device 100 further includes a first limiting member 61 and a second limiting member 62 received in the receiving groove 106. The first limiting member 61 and the second limiting member 62 are configured to limit opposite ends of the first conductive member 41, respectively. Optionally, the first conductive member 41 includes a main body 411, a first hook portion 412, and a second hook portion 413. The main body 411 covers part of the opening of the receiving groove 106 or cover the entire opening of the receiving groove 106.

Optionally, refer to FIG. 6 and FIG. 8, the first hook portion 412 may be the same as the second hook portion 413 in structure, and the first limiting member 61 may be the same as the second limiting member 62 in structure. Specifically, both the first hook portion 412 and the second hook portion 413 are hook-shaped, and the first hook portion 412 and the second hook portion 413 are mirror-symmetrically arranged at opposite ends of the main body 411. Both the first limiting member 61 and the second limiting member 62 may be pins. Both the first limiting member 61 and the second limiting member 62 extend in the Z-axis direction. The receiving groove 106 defines inserting holes 63 and 64 on a groove wall, where the inserting hole 63 matches the first limiting member 61, and the inserting hole 64 matches the second limiting member 62. The first conductive member 41 may be mounted in the receiving groove 106 from the external space 400, the inserting hole 63 faces a hook space of the first hook portion 412, and the inserting hole 64 faces a hook space of the second hook portion 413. Here, the first limiting member 61 and the second limiting member 62 are respectively inserted into the inserting hole 63 and the inserting hole 64 in the Z-axis direction from a side of the housing frame 1.

Optionally, since the first hook portion 412 and the second hook portion 413 protrude from the main body 411, grooves may be defined on the bottom surface of the receiving groove 106 at positions corresponding to the first hook portion 412 and the second hook portion 413. A gap may be defined between the bottom surface of one of the grooves and the first hook portion 412, and another gap may be defined between the bottom surface of the other groove and the second hook portion 413, so that a distance between the bottom surface of the receiving groove 106 and the outer surface 105 of the housing frame 1 may be shortened. In this way, a thickness of the housing frame 1 in the X-axis direction can be reduced.

Optionally, refer to FIG. 6 and FIG. 7, a size of the first conductive member 41 in the Y-axis direction is much larger than a size of the first through hole 103 in the Y-axis direction. The electronic device 100 further includes a first elastic member 65 and a second elastic member 66 that are at least partially received in the receiving groove 106. The first elastic member 65 and the second elastic member 66 are arranged at opposite sides of the first through hole 103, respectively. Both the first elastic member 65 and the second elastic member 66 elastically abut against a bottom of the receiving groove 106 and the first conductive member 41. Both the first elastic member 65 and the second elastic member 66 may be in a compressed state. The first elastic member 65 and the second elastic member 66 may provide sufficient elastic support forces at opposite sides of the first through hole 103, so that any part of the first conductive member 41 in the Y-axis direction may be mounted in a stable state. At the same time, when the first conductive member 41 is pressed, the first elastic member 65 and the second elastic member 66 may provide enough elastic recovery forces, so that when the pressing force exerted on the first conductive member 41 is removed, the first conductive member 41 may react quickly and recover to original location.

In this way, the first elastic member 65 and the second elastic member 66 may exert elastic forces on the first conductive member 41 in a direction toward the external space 40, and the first limiting member 61 and the second limiting member 62 may exert restraining forces on the first conductive member 41 in a direction toward the inner cavity 102, so that the first conductive member 41 can be in a state of force balance in the receiving groove 106. At the same time, there are a distance between the first hook portion 412 and the bottom surface of the receiving groove 106 and a distance between the second hook portion 413 and the bottom surface of the receiving groove 106, and there are a distance between the first limiting member 61 and the main body 411 and a distance between the second limiting member 62 and the main body 411, and thus the first elastic member 65 and the second elastic member 66 may be further compressed, so that when the first conductive member 41 is subjected to a pressed force toward the inner cavity 102 of the housing frame 1, the first conductive member 41 may further press the first elastic member 65 and the second elastic member 66, and in turn further press the second conductive member 42 to trigger the switch unit 53.

When the first conductive member 41 is pressed by an external pressure, the first elastic member 65, an elastic conductive member 7, and the second elastic member 66 are compressed, the first conductive member 41 moves toward the bottom of the receiving groove 106, and the first conductive member 41 pushes the second conductive member 42 to move toward the switch unit 53 to trigger the switch unit 53. When the external pressure exerted on the first conductive member 41 is removed, the first conductive member 41 moves away from the bottom of the receiving groove 106 under elastic recovery forces of the first elastic member 65, the elastic conductive member 7, and the second elastic member 66 until the first hook portion 412 abuts against the first limiting member 61 and the second hook portion 413 abuts against the second limiting member 62.

Further, the first elastic member 65 and the second elastic member 66 may be arranged close to the first hook portion 412 and the second hook portion 413, respectively, so that mounting stability of the first conductive member 41 can improved, and problems such as unstable mounting and shaking can be prevented.

Structures and materials of the first elastic member 65 and the second elastic member 66 are not limited herein. Optionally, the first elastic member 65 may be the same as the second elastic member 66 in structure. For example, the first elastic member 65 and the second elastic member 66 are both springs. Optionally, the first elastic member 65 and the second elastic member 66 may be elastic silicone, metal shrapnel, plastic shrapnel, etc.

Further, refer to FIG. 6 and FIG. 8, one end of the first elastic member 65 and one end of the second elastic member 66 may be fixed on the bottom surface of the receiving groove 106, so that the first elastic member 65 and the second elastic member 66 may be better fixed in the receiving groove 106. The main body 411 may define a groove 107 and a groove 108 on a side surface of the main body 411 facing the bottom surface of the receiving groove 106, and the groove 107 corresponds to the first elastic member 65 and the groove 108 corresponds to the second elastic member 66, so that part of the first elastic member 65 and part of the second elastic member 66 may be accommodated in the groove 107 and the groove 108 in the main body 411, respectively, and thus the first elastic member 65 and the second elastic member 66 may be positioned, and the thickness of the housing frame 1 in the X-axis direction may be further reduced.

The elastic abutment between the first conductive member 41 and the second conductive member 42 includes but is not limited to the following two cases. In one case, at least one of the first conductive member 41 or the second conductive member 42 is made of an elastic conductive material, and the first conductive member 41 is in direct contact with the second conductive member 42. In another case, the elastic conductive member 7 is provided between the first conductive member 41 and the second conductive member 42, and the elastic conductive member 7 elastically abuts against the first conductive member 41 and the second conductive member 42.

Implementations of the elastic abutment between the first conductive member 41 and the second conductive member 42 are described hereinafter in conjunction with the accompanying drawings. The elastic abutment between the first conductive member 41 and the second conductive member 42 in the implementations of the disclosure includes but is not limited to the following implementations.

In a first possible implementation, refer to FIGs. 6-8, the first conductive member 41 is connected with the second conductive member 42 via the elastic conductive member 7, and the first conductive member 41 may elastically abut the second conductive member 42, so that the first conductive member 41 and the second conductive member 42 is movable relative to each other.

Specifically, the elastic conductive member 7 includes but is not limited to a spring, a metal shrapnel, a plastic shrapnel, an elastic conductive silica gel, an elastic conductive rubber, an elastic conductive foam, etc.

The first conductive member 41 may be spaced apart from the second conductive member 42, and the first conductive member 41 can move relative to the second conductive member 42. The elastic conductive member 7 elastically abuts against the first conductive member 41 and the second conductive member 42. The first conductive member 41 squeezes the elastic conductive member 7 under an external force to compress the elastic conductive member 7. The first conductive member 41 gradually approaches the second conductive member 42 during compression of the elastic conductive member 7. The elastic conductive member 7 may realize both the electrical connection and elastic buffering between the first conductive member 41 and the second conductive member 42.

The first conductive member 41 may be slidably connected to the second conductive member 42, and the first conductive member 41 can move relative to the second conductive member 42. The first conductive member 41 squeezes the elastic conductive member 7 under an external force, and the first conductive member 41 gradually slides toward the second conductive member 42 during compression of the elastic conductive member 7. The elastic conductive member 7 may realize both the electrical connection and elastic buffering between the first conductive member 41 and the second conductive member 42.

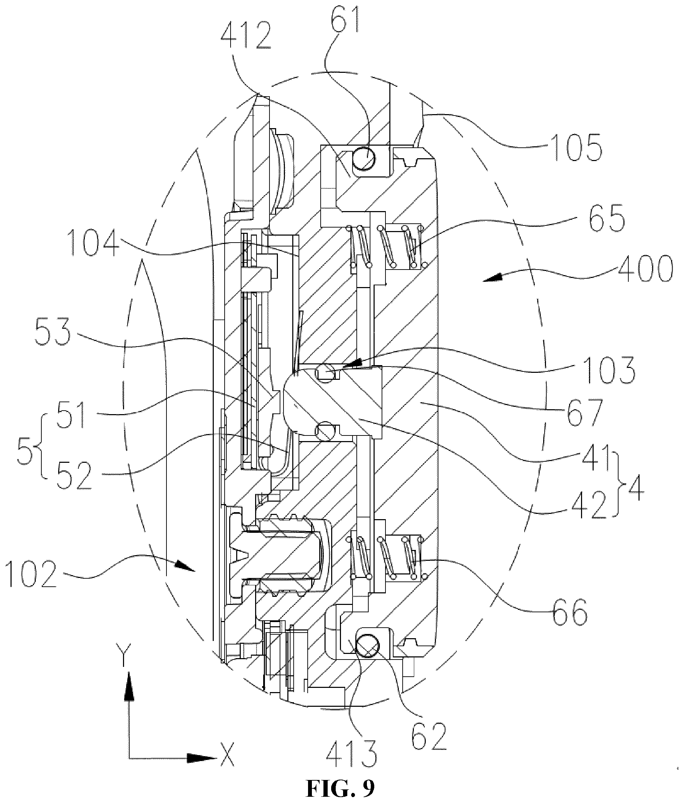

In a second possible implementation, refer to FIG. 9, the first conductive member 41 abuts against the second conductive member 42, and at least one of the first conductive member 41 or the second conductive member 42 is made of an elastic conductive material. For example, at least one of the first conductive member 41 or the second conductive member 42 is made of elastic conductive rubber, elastic conductive silicone, or the like. Optionally, the first conductive member 41 is made of an elastic conductive material, the second conductive member 42 is made of a metal conductive material. When receiving an impact force, the first conductive member 41 is compressed to absorb the impact force, thereby reducing an impact transmitted to the circuit assembly 5 and avoiding damage to structures on a force transmission path. Optionally, the second conductive member 42 is made of an elastic conductive material, and the first conductive member 41 is made of a metal conductive material. Optionally, the second conductive member 42 is made of an elastic conductive material, and the first conductive member 41 is made of an elastic conductive material. The above implementations may all reduce impact.

The first conductive member 41 is connected with the second conductive member 42 via the elastic conductive member 7, which includes but is not limited to the following implementations.

In a first implementation, refer to FIG. 7, the elastic conductive member 7 includes a conductive retractable member 71. One end of the conductive retractable member 71 is connected to the first conductive member 41 thought a connection manner such as direct abutment, bonding via a conductive adhesive, soldering or welding, press-fitting, or engagement via a conductive engaging member. The other end of the conductive retractable member 71 is connected to the second conductive member 42 by direct abutment, bonding via a conductive adhesive, soldering or welding, press-fitting, or engagement via a conductive engaging member. The conductive retractable member 71 includes but is not limited to a conductive spring, a conductive resilient piece, a conductive silica gel, or the like.

Specifically, one end of the second conductive member 42 may be in the receiving groove 106 and abut against the first conductive member 41 via the conductive retractable member 71, and the first conductive member 41 may be spaced apart from the second conductive member 42. The conductive retractable member 71 is disposed between the first conductive member 41 and the second conductive member 42. The second conductive member 42 extends through the first through hole 103, and the other end of the second conductive member 42 extends into the inner cavity 102 and abuts against the electrical contact portion 52. When the first conductive member 41 pushes the conductive retractable member 71 to compress, the conductive retractable member 71 pushes the second conductive member 42 to move in the second through hole and press the electrical contact portion 52, so that the above-identified biometric feature detection can be carried out or the switch unit 53 can be switched on.

The second conductive member 42 may be columnar, and specifically, may be cylindrical, square columnar, etc., which is not limited herein. A radial size of the second conductive member 42 may be slightly smaller than a radial size of the first through hole 103.

In an optional implementation, refer to FIG. 6, the second conductive member 42 defines a first groove 110 at one end of the second conductive member 42 facing the first conductive member 41. Part of the conductive retractable member 71 is received in the first groove 110 and abuts or is fixedly connected to a bottom of the first groove 110. In this way, on the one hand, positioning of the conductive retractable member 71 can be realized, on the other hand, the second conductive member 42 defines a space for accommodating the conductive retractable member 71, and with the space, it is unnecessary to reserve a space between the first conductive member 41 and the second conductive member 42 to accommodate the compressed conductive retractable member 71, in other words, the conductive retractable member 71 may be accommodated in the first groove 110 after being compressed, so that a distance between the first conductive member 41 and the second conductive member 42 may be utilized for pressure absorbing or impact absorbing, and thus an overall assembly thickness of the first conductive member 41, the second conductive member 42, and the conductive retractable member 71 in the X-axis direction can be reduced.

In an optional implementation, refer to FIG. 6, the first conductive member 41 defines a second groove 111 on a side surface of the first conductive member 41 facing the second conductive member 42. The other end of the conductive retractable member 71 abuts against or is fixedly connected to a bottom of the second groove 111. The implementation in which the second groove 111 is defined may be combined with the implementation in which the first groove 110 is defined at the one end of the second conductive member 42 facing the first conductive member 41. The opening of the second groove 111 communicates with the opening of the first groove 110, so that the other end of the conductive retractable member 71 can be guided and positioned. Part of the conductive retractable member 71 may be received in the second groove 111, and another part of the conductive retractable member 71 may be received in the first groove 110. The first groove 110 and the second groove 111 cooperatively allow the conductive retractable member 71 to have a relatively long length on premise of a relatively small distance between the first conductive member 41 and the second conductive member 42.

Optionally, refer to FIG. 6 and FIG. 7, the electronic device 100 further includes a sealing ring 67. The sealing ring 67 may be made of a material with good sealing performance such as rubber, silicone, which is not limited herein. The sealing ring 67 is sleeved on a peripheral side wall of the second conductive member 42. An outer contour surface of the sealing ring 67 closely contacts a hole wall of the first through hole 103, and the sealing ring 67 may be slidable relative to the first through hole 103. An inner contour surface of the sealing ring 67 closely contacts the peripheral side wall of second conductive member 42. The sealing ring 67 is made of a waterproof material, and the sealing ring 67 is sealingly connected between the peripheral side wall of the second conductive member 42 and the hole wall of the first through hole 103, so that a waterproof between the second conductive member 42 and the housing frame 1 can be realized, and thus it is possible to prevent water from entering the circuit assembly 5 through the first through hole 103.

Further, the second conductive member 42 defines an annular groove 68 on the outer peripheral surface of the second conductive member 42, and the annular groove 68 is used for accommodating the sealing ring 67, realizing a positioning of the sealing ring 67. In addition, the sealing ring 67 is compressed between the second conductive member 42 and the hole wall of the first through hole 103. The second conductive member 42 and the sealing ring 67 are in an interference fit with the hole wall of the first through hole 103, so that 5 atmospheres (5 ATM) waterproof requirement can be met, that is, the electronic device 100 meets the atmospheric pressure waterproof requirement of 5 ATMs.

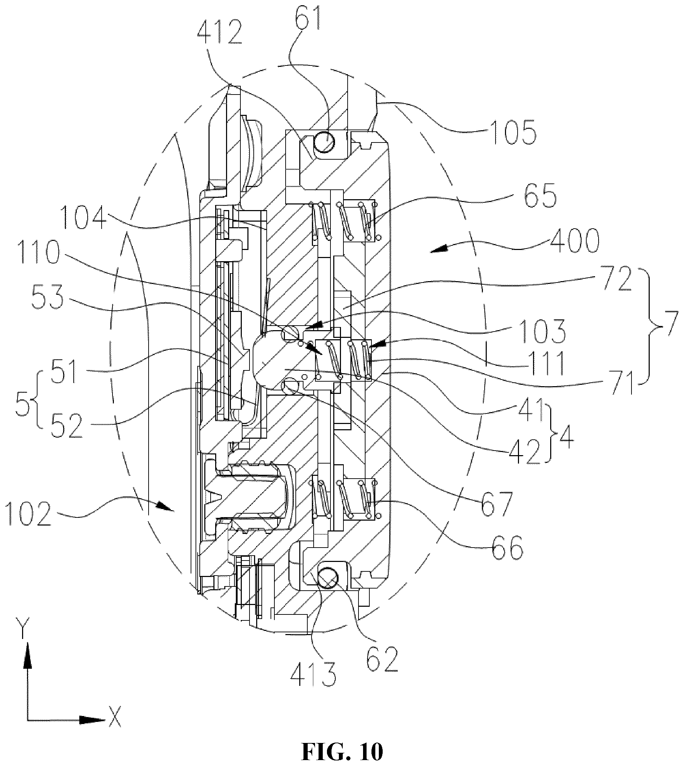

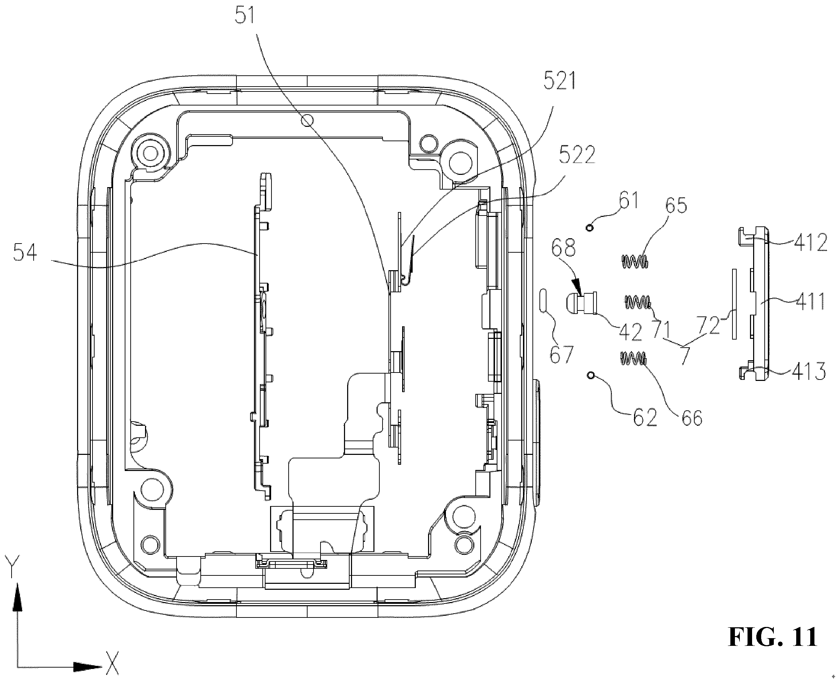

Refer to FIG. 10 and FIG. 11, a second implementation is the same as the first implementation, except that the elastic conductive member 7 is further provided with an elastic gasket 72. The elastic gasket 72 abuts against the first conductive member 41 and the second conductive member 42. The elastic gasket 72 may be made of a material that includes but is not limited to elastic silicone, elastic rubber, elastic polymer, and the like. The elastic gasket 72 may be conductive or non-conductive.

Refer to FIG. 10 and FIG. 11, the elastic gasket 72 is disposed between the first conductive member 41 and the second conductive member 42. With aid of a compressibility of the elastic gasket 72, a tolerance zone can be absorbed in case of manufacturing errors in the first conductive member 41 and the second conductive member 42, to ensure stable hand-feel of the button assembly 4 in a mass production. It is also possible to ensure that the elastic gasket 72 is not easy to deform under extreme operating conditions in comparison with springs, etc., so that an impact force can be absorb to a greater extent, and a buffering can be provided for the button assembly 4 in collision/dropping conditions, thereby avoiding failure of the button assembly 4 in collision/dropping conditions, and prolonging a service life.

Further, the elastic gasket 72 is in a compressed state. In short, the elastic gasket 72 is in an interference fit between the first conductive member 41 and the second conductive member 42. That is, in the X-axis direction, the elastic gasket 72 is squeezed between the first conductive member 41 and the second conductive member 42. The elastic gasket 72 exerts a squeezing resistance to the first conductive member 41 in a positive direction of X-axis and a squeezing resistance to the second conductive member 42 in a negative direction of X axis. As such, the elastic gasket 72 may support the first conductive member 41, thereby preventing the button assembly 4 from shaking, and improving stability of the button assembly 4.

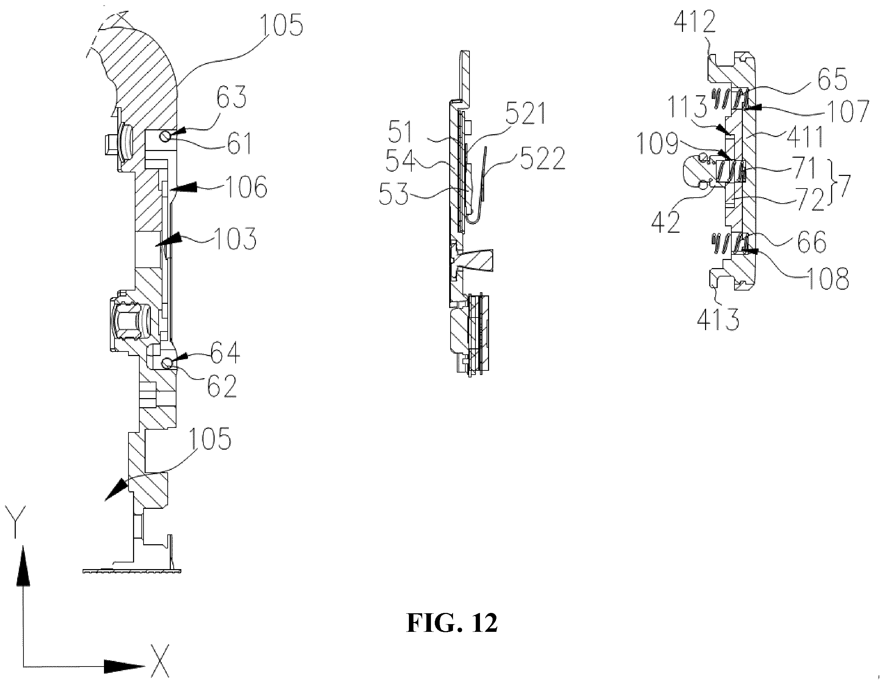

Specifically, refer to FIG. 12, the elastic gasket 72 and the conductive retractable member 71 may in a sleeve connection with each other in the X-axis direction. Optionally, the elastic gasket 72 is sleeved on an outer periphery of the conductive retractable member 71. Optionally, the conductive retractable member 71 is sleeved on an outer periphery of the elastic gasket 72. In this way, an overall size of the elastic gasket 72 and the conductive retractable member 71 in the X-axis direction is reduced, thereby making the elastic conductive member 7, the first conductive member 41, and the second conductive member 42 more compact in structure. In the implementations, the elastic gasket 72 is disposed at a side of the first conductive member 41 facing the second conductive member 42. The first conductive member 41 defines a third groove 113 on a side surface of the first conductive member 41 facing the second conductive member 42. The second groove 111 is defined on a bottom surface of the third groove 113. The elastic gasket 72 is received in the third groove 113, on the one hand, the third groove 113 provides a space for positioning and accommodating the elastic gasket 72, and on the other hand, the elastic gasket 72 is disposed in the first conductive member 41, so that an overall size of the elastic gasket 72 and the first conductive member 41 in the X-axis direction can be reduced, and the compactness of the button assembly 4 can be improved.

Refer to FIG. 10, a side surface of the elastic gasket 72 facing the second conductive member 42 may be spaced apart from the bottom surface of the receiving groove 106. An orthographic projection of the elastic gasket 72 on a side where the second conductive member 42 is located may cover the first through hole 103, so that a sufficiently large impact-absorption area can be provided and a relatively large support area can be provided for the first conductive member 41. The other end of the second conductive member 42 extending into the receiving groove 106 may abut against the elastic gasket 72, so that the elastic gasket 72 is compressed between the first conductive member 41 and the second conductive member 42.

Refer to FIG. 12, the elastic gasket 72 defines a second through hole 109 communicating with the first through hole 103. Refer to FIG. 10, the second through hole 109 may communicate with the first groove 110 and the second groove 111. The one end of the conductive retractable member 71 abuts against or is fixedly connected to the bottom of the first groove 110. The conductive retractable member 71 extends through the second through hole 109. The other end of the conductive retractable member 71 abuts or is fixedly connected to the bottom of the second groove 111. In this way, the conductive retractable member 71 extends through the second through hole 109 to reduce an overall size of the conductive retractable member 71 and the elastic gasket 72 in the X-axis direction.

An expansion and contraction of the conductive retractable member 71 in the X-axis direction may be independent of an expansion and contraction of the elastic gasket 72 in the X-axis direction. In this way, both the conductive retractable member 71 and the elastic gasket 72 may provide elastic support for the first conductive member 41, so that the button assembly 4 may be stably mounted.

In an example, the first conductive member 41 serves as the button cap and the second conductive member 42 serves as the button post, and assembly relationships among various components in the implementations are illustrated as follows. First, mount (for example, paste) the flexible circuit board 51 on the circuit board support 54; mount the circuit board support 54 on an inner side of the housing frame 1; pre-sleeve the sealing ring 67 in the annular groove 68 of the second conductive member 42; mount the second conductive member 42 in the first through hole 103 of the housing frame 1, and make one end of the second conductive member 42 abut against the electrical contact portion 52 of the flexible circuit board 51; mount the conductive retractable member 71 in the first groove 110 of the button post; mount the first elastic member 65 and the second elastic member 66 in the receiving groove 106, and pre-mount the elastic gasket 72 in the third groove 113 of the first conductive member 41; mount the first conductive member 41 in the receiving groove 106 of the housing frame 1, so that one end of the button post abuts against the elastic gasket 72 and the conductive retractable member 71 abuts against the bottom of the second groove 111 of the first conductive member 41; pre-press the first conductive member 41 to make the hook space of the hook portion of the first conductive member 41 face a corresponding inserting hole, insert the first limiting member 61 and the second limiting member 62 into the first inserting hole 63 and the second inserting hole 64, respectively, and press the first limiting member 61 and the second limiting member 62 to ends, thereby completing assembly.

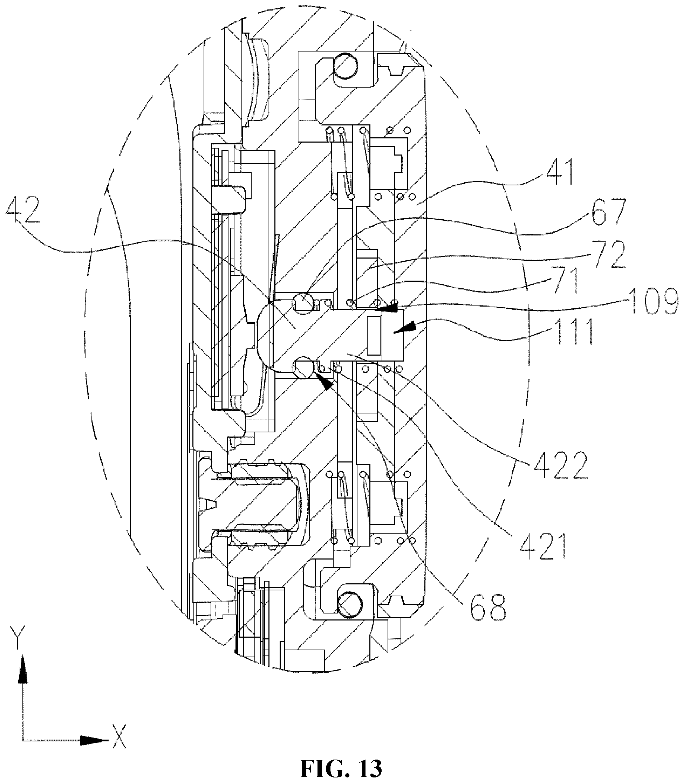

In a third implementation, refer to FIG. 13, the conductive retractable member 71 may be in a shape of a ring. Specifically, the conductive retractable member 71 may be a spring. Different from the second implementation, in the third implementation, part of the conductive retractable member 71 is sleeved on the peripheral side wall of the second conductive member 42, and the peripheral side wall of the second conductive member 42 faces the hole wall of the first through hole 103, so that a contact area between the conductive retractable member 71 and the second conductive member 42 can be increased, and an electrical connection reliability between the conductive retractable member 71 and the second conductive member 42 can be improved. When the conductive retractable member 71 is sleeved on the peripheral side wall of the second conductive member 42, one end of the conductive retractable member 71 may be fixedly connected or slidably connected to the peripheral side wall of the second conductive member 42.

Further, refer to FIG. 13, the second conductive member 42 is provided with an engaging post 421 on the peripheral side wall of the second conductive member 42. The engaging post 421 extends in a radial direction. One end of the conductive retractable member 71 is engaged with the engaging post 421. Optionally, the engaging post 421 may be in a shape of a ring and disposed on the peripheral side wall of the second conductive member 42. The conductive retractable member 71 may be a helical spring. In this way, part of helical coils of the conductive retractable member 71 may be sleeved on the engaging post 421, so that the conductive retractable member 71 may be connected to the engaging post 421, and thus the conductive retractable member 71 can be detachably connected to the engaging post 421 without additional connections, which is convenient for mounting.

The other end of the conductive retractable member 71 abuts against or is fixedly connected to the first conductive member 41. Specifically, the other end of the conductive retractable member 71 may be fixedly connected to the first conductive member 41 in a connection manner such as bonding via a conductive adhesive, soldering or welding, and press-fitting.

It is noted that the one end of the conductive retractable member 71 in the implementation illustrated in FIG. 13 is not limited to be the same as the one end of the conductive retractable member 71 in the first implementation. In other words, the one end of the conductive retractable member 71 in the implementation illustrated in FIG. 13 may be the other end of the conductive retractable member 71 in the first implementation, or the one end of the conductive retractable member 71 in the first implementation.

Further, the engaging post 421 in the implementations may be formed as follows. The second conductive member 42 defines the annular groove 68 on the peripheral side wall of the columnar second conductive member 42 and has a thinned cylinder spaced apart from the annular groove 68 in the X-axis direction, and a portion of the second conductive member 42 between the annular groove 68 and the thinned cylinder serves as the engaging post 421. The annular groove 68 is used for accommodating the sealing ring 67. The conductive retractable member 71 is sleeved on the outer peripheral surface of the thinned cylinder, and the one end of the conductive retractable member 71 is connected to the first conductive member 41.

Further, refer to FIG. 13, the first conductive member 41 defines the second groove 111 on the side surface of the first conductive member 41 facing the second conductive member 42. One end of the second conductive member 42 is in the second groove 111 and spaced from the bottom of the second groove 111. The peripheral side wall of the second conductive member 42 is at least partially slidably connected to a groove wall of the second groove 111. When the conductive retractable member 71 is pressed, the second conductive member 42 is at least partially retractable in the second groove 111. The one end of the second conductive member 42 may directly contact the groove wall of the second groove 111 of the first conductive member 41. The second conductive member 42 is kept in connect with the first conductive member 41, so that stability of an electrical contact between the second conductive member 42 and the first conductive member 41 can be improved.

Optionally, there may be no elastic gasket 72 provided between the first conductive member 41 and the second conductive member 42.

Optionally, refer to FIG. 13, the elastic gasket 72 may be disposed between the first conductive member 41 and the second conductive member 42. Specifically, the elastic gasket 72 defines the second through hole 109, the second through hole 109 communicates with the second groove 111, and the second conductive member 42 extends through the second through hole 109. Optionally, the conductive retractable member 71 may extend through the second through hole 109, that is, the second conductive member 42, the conductive retractable member 71, and the elastic gasket 72 are arranged sequentially from inside to outside in the Y-axis direction. Optionally, the conductive retractable member 71 may be connected to the elastic gasket 72, so that impact absorption capabilities of the elastic gasket 72 and the conductive retractable member 71 can be combined in the X-axis direction. Specifically, the conductive retractable member 71 is a spring, and the conductive retractable member 71 may extend through the elastic gasket 72, or the elastic gasket 72 is clamped between two adjacent coils of the conductive retractable member 71, so that the elastic gasket 72 is fixed with the conductive retractable member 71, stability of a connection between the elastic gasket 72 and the conductive retractable member 71 is improved. Moreover, the conductive retractable member 71 and the elastic gasket 72 are stacked in the X-axis direction, so that the impact absorption capabilities of the elastic gasket 72 and the conductive retractable member 71 are combined in the X-axis direction.

The conductive retractable member 71 may extend through the elastic gasket 72. Alternatively, the elastic gasket 72 may be clamped between two adjacent coils of the conductive retractable member 71. New implementations may also be obtained by suitably combining the implementation with other implementations of the disclosure.

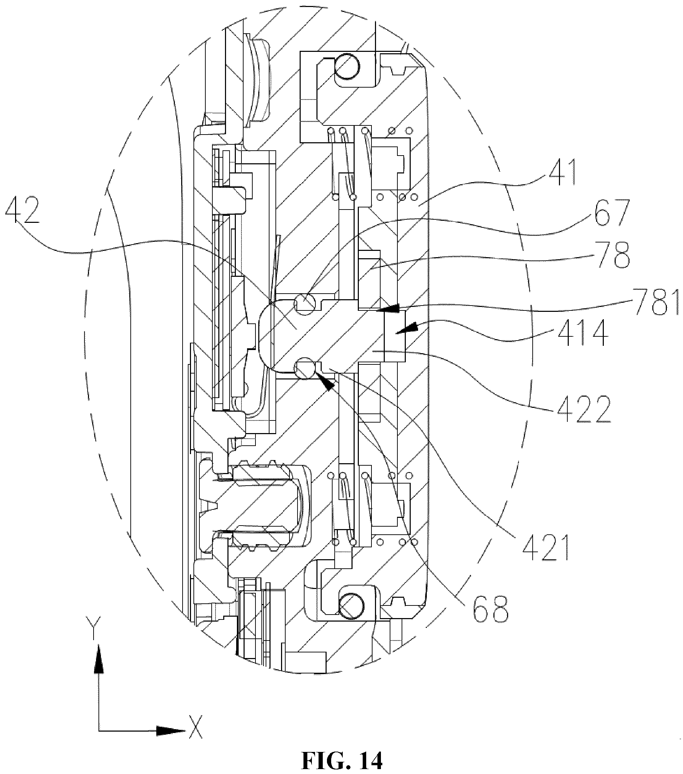

Refer to FIG. 14, a fourth implementation is the same as the third implementation, except that in the fourth implementation, there is no conductive retractable member 71 that is provided in the third implementation, instead that an elastic structure 78 is provided. The elastic structure 78 provides an elastic supporting force and an elastic restoring force between the first conductive member 41 and the engaging post 421 of the second conductive member 42.

Refer to FIG. 14, the elastic structure 78 defines a third through hole 781. The elastic structure 78 may be a spring or an elastic gasket.

Refer to FIG. 14, the second conductive member 42 may be provided with a protrusion portion 422 on a side surface of the second conductive member 42 facing the first conductive member 41. The first conductive member 41 may define a groove portion 414 on the side surface of the first conductive member 41 facing the second conductive member 42, and the groove portion 414 faces the protrusion portion 422. The groove portion 414 communicates with the third through hole 781. The protrusion portion 422 extends through the third through hole 781. An end surface of the protrusion portion 422 faces and is spaced apart from a bottom wall of the groove portion 414. A peripheral side wall of the protrusion portion 422 is slidably connected to a peripheral side wall of the groove portion 414, so that the first conductive member 41 and the second conductive member 42 are slidably connected in the X-axis direction. The first conductive member 41 may kept in contact with the second conductive member 42, and the first conductive member 41 is in electrical communication with the second conductive member 42, so that stability of an electrical connection between the first conductive member 41 and the second conductive member 42 is improved, and the elastic structure does not need to be conductive. In addition, positions of the protrusion portion 422 and the groove portion 414 may be interchanged.

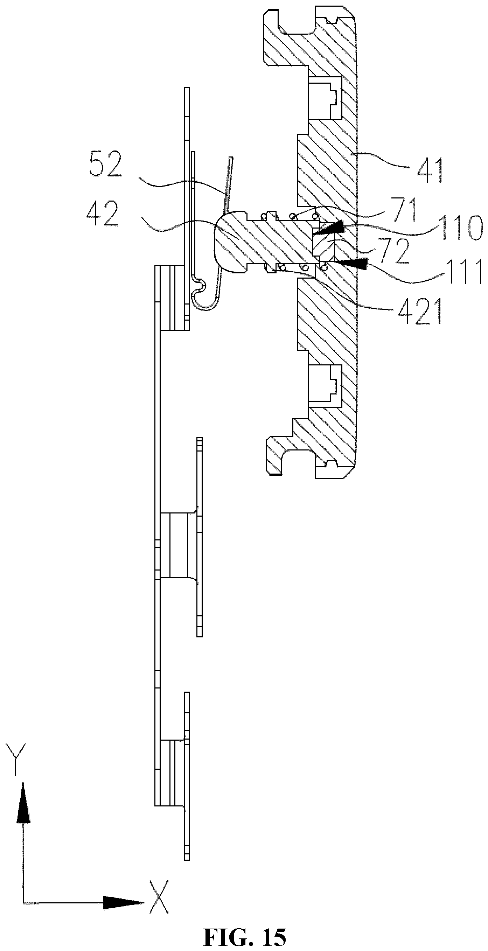

Refer to FIG. 15, a fifth implementation is substantially the same as the third implementation, where the conductive retractable member 71 is in a shape of a ring, and the conductive retractable member 71 abuts against the first conductive member 41 and the second conductive member 42, and the like. The fifth implementation differs from the third implementation in that the elastic gasket 72 extends through an axial hole of the conductive retractable member 71. Specifically, the elastic gasket 72 is compressed in the second groove 111 of the first conductive member 41 and the first groove 110 of the second conductive member 42. In this way, the elastic gasket 72 does not need to be disposed on the outer periphery of the conductive retractable member 71, and an internal space of the conductive retractable member 71 can be fully utilized by the elastic gasket 72, thereby improving compactness of an arrangement of the elastic gasket 72 and the conductive retractable member 71.

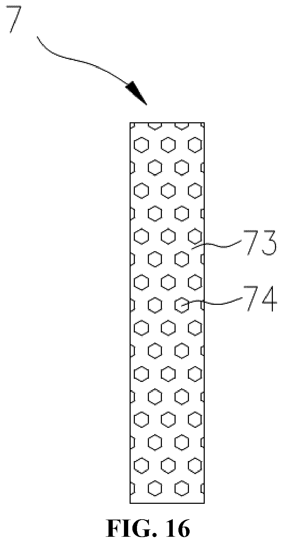

Refer to FIG. 16, in a sixth implementation, the elastic conductive member 7 includes an elastic substrate 73 and multiple conductive base-material-portions 74. The multiple conductive base-material-portions are doped in the elastic substrate 73. The multiple conductive base-material-portions 74 make the elastic conductive member 7 electroconductive at least in the X-axis direction. The elastic conductive member 7 may be a conductive rubber gasket. The elastic base material may be a rubber gasket, a silicone gasket, polyvinyl alcohol elastomer, or other elastomers. The conductive base-material-portions 74 may be conductive metal particles, conductive metal wires, conductive metal wires, conductive metal grids, graphene, and the like. The conductive base-material-portions 74 may be a conductive metal such as copper, gold, silver, aluminum, and the like.

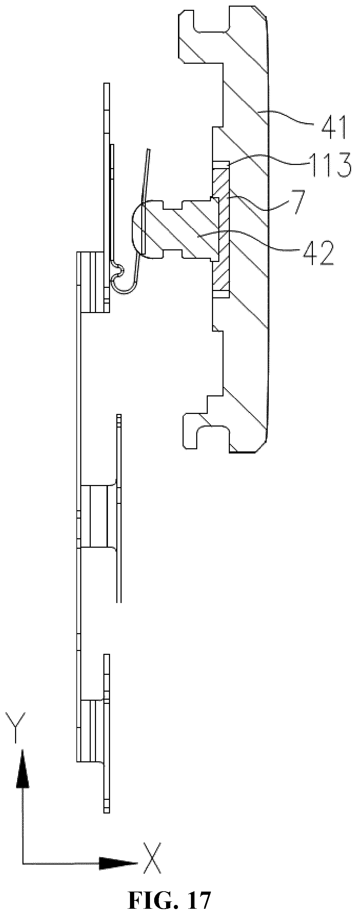

Refer to FIG. 17, the elastic conductive member 7 having elasticity and conductivity is disposed in the third groove 113 of the first conductive member 41, so that elastic abutment and electrical connection between the first conductive member 41 and the second conductive member 42 can be realized. The elastic conductive member 7 provided in the implementations may be a block, which occupies a small space, is easy to mount, and has good stability.

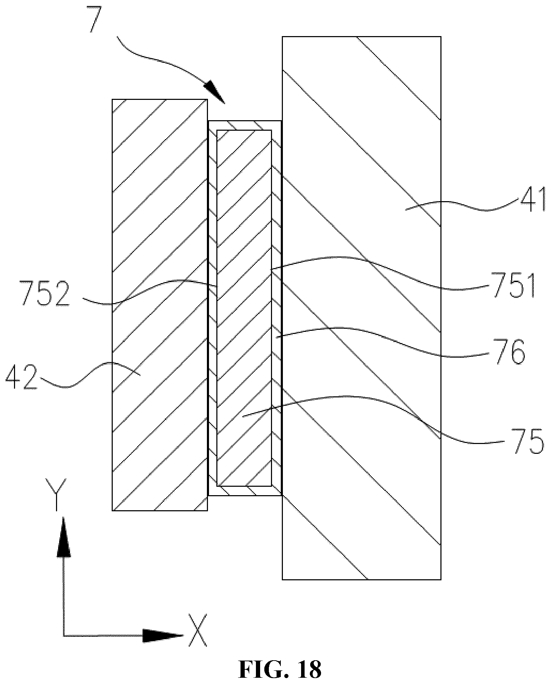

Refer to FIG. 18, in a seventh implementation, the elastic conductive member 7 further includes an elastic main body 75 and a conductive covering portion 76. The elastic main body 75 may be a rubber gasket, a silicone gasket, a polyvinyl alcohol elastomer, or other elastomers.

Refer to FIG. 18, the elastic main body 75 includes a first face 751 and a second face 752 opposite the first face 751. A part of the conductive covering portion 76 is disposed on the first face 751 and connected to the first conductive member 41. Another part of the conductive covering portion 76 is disposed on the second face 752 and connected to the second conductive member 42. The conductive covering portion 76 may be a conductive metal layer, a conductive metal foil, and the like. The conductive covering portion 76 may be made of a conductive metal such as copper, gold, silver, aluminum, and the like.

Specifically, the first face 751 of the elastic main body 75 abuts against the first conductive member 41, so that a part of the conductive covering portion 76 contacts the first conductive member 41. The second face 752 of the elastic main body 75 abuts against the second conductive member 42, so that another part of the conductive covering portion 76 contacts the second conductive member 42. The part of the conductive covering portion 76 contacting the first conductive member 41 is electrically connected to the other part of the conductive covering portion 76 contacting the second conductive member 42. In this way, the elastic abutment and electrical connection between the first conductive member 41 and the second conductive member 42 are realized. The conductive covering portion 76 provided in the implementations is a thin layer, so that the elastic conductive member 7 occupies a small space, is easy to mount, and has good stability.

Further, the conductive covering portion 76 may be a flexible conductive film. The flexible conductive film has scalability to adapt to the extension and retraction of the elastic main body 75. The flexible conductive film may be a stretchable adhesive layer doped with the conductive base-material-portions 74. The conductive base-material-portions 74 may be conductive metal particles, conductive metal wires, conductive metal wires, conductive metal grids, graphene, and the like. The conductive base-material-portion 74 may be made of a conductive metal such as copper, gold, silver, aluminum, or the like. The conductive covering portion 76 may be wrapped on part of an outer peripheral surface of the elastic main body 75 or wrapped on the entire outer peripheral surface of the elastic main body 75, or the conductive covering portion 76 may be wrapped around the outer peripheral surface of the elastic main body 75 in a spiral manner, so that the conductive covering portion 76 may adapt to the extension and retraction of the elastic main body 75 without being broken.

The above implementations are exemplary implementations of the disclosure, and it is noted that various improvements and modifications may be made without departing from the principle of the disclosure to those of ordinary skill in the art, and the improvements and the modifications are also considered as the protection scope of the disclosure.

## Claims

1. An electronic device, comprising:
a housing frame having an inner cavity and a first through hole, wherein the first through hole communicates with the inner cavity and an external space outside the housing frame;
a button assembly comprising a first conductive member and a second conductive member, wherein the first conductive member is connected to the housing frame and at least partially in the external space, the second conductive member is at least partially in the first through hole, and the first conductive member elastically abuts against the second conductive member, so that the first conductive member is in electrical communication with the second conductive member; and
a circuit assembly disposed in the inner cavity, wherein the circuit assembly comprises a circuit board and an electrical contact portion electrically connected to the circuit board, and the electrical contact portion abuts against an end of the second conductive member away from the first conductive member so that the first conductive member is in electrical communication with the electrical contact portion.

2. The electronic device of claim 1, wherein the button assembly further comprises an elastic conductive member, and wherein the elastic conductive member elastically abuts between the first conductive member and the second conductive member, so that the first conductive member and the second conductive member are movable relative to each other.

3. The electronic device of claim 2, wherein the elastic conductive member comprises a conductive retractable member, and wherein the conductive retractable member has one end connected to the first conductive member and the other end connected to the second conductive member.

4. The electronic device of claim 3, wherein the second conductive member defines a first groove at one end of the second conductive member facing the first conductive member, and a part of the conductive retractable member is received in the first groove, and abuts against or is fixedly connected to a bottom of the first groove.

5. The electronic device of claim 3, wherein the first conductive member defines a second groove on a side surface of the first conductive member facing the second conductive member, and another part of the conductive retractable member is received in the second groove, and abuts against or is fixedly connected to a bottom of the second groove.

6. The electronic device of claim 3, wherein the first conductive member defines a second groove on a side surface of the first conductive member facing the second conductive member, and the second conductive member is at least partially received in the second groove when the conductive retractable member is pressed.

7. The electronic device of claim 3, wherein the conductive retractable member is in a shape of a ring, and the conductive retractable member is at least partially sleeved on a peripheral side wall of the second conductive member.

8. The electronic device of claim 7, wherein the second conductive member is provided with an engaging post on the peripheral side wall of the second conductive member, and wherein the one end of the conductive retractable member is engaged with the engaging post, and the other end of the conductive retractable member abuts against or is fixedly connected to the first conductive member.

9. The electronic device of any one of claims 3 to 8, wherein the elastic conductive member further comprises an elastic gasket abutting between the first conductive member and the second conductive member, and wherein the elastic gasket is sleeved on an outer periphery of the conductive retractable member, or the elastic conductive member is sleeved on an outer periphery of the elastic gasket.

10. The electronic device of any one of claims 3 to 8, wherein
the elastic conductive member further comprises an elastic gasket, wherein the elastic gasket abuts against at least one of the first conductive member or the second conductive member; and
the conductive retractable member is a spring, wherein the conductive retractable member extends through the elastic gasket, or the elastic gasket is clamped between two adjacent coils of the conductive retractable member.

11. The electronic device of claim 2, wherein the elastic conductive member comprises an elastic substrate and a plurality of conductive base-material-portions doped in the elastic substrate, and wherein the plurality of conductive base-material- portions make the elastic conductive member electroconductive.

12. The electronic device of claim 2, wherein the elastic conductive member further comprises an elastic main body and a conductive covering portion, wherein the elastic main body has a first face and a second face opposite the first face, and wherein a part of the conductive covering portion is disposed on the first face and connected to the first conductive member, and another part of the conductive covering portion is disposed on the second face and connected to the second conductive member.

13. The electronic device of claim 12, wherein the conductive covering portion is a flexible conductive film, and wherein the conductive covering portion is wrapped on an outer peripheral surface of the elastic main body, or the conductive covering portion wraps around the outer peripheral surface of the elastic main body in a spiral shape.

14. The electronic device of claim 1, wherein the first conductive member abuts against the second conductive member, and at least one of the first conductive member or the second conductive member is made of an elastic conductive material.

15. The electronic device of claim 2, wherein
the elastic conductive member defines a second through hole, the button assembly further comprises a protrusion portion and a groove portion facing the protrusion portion, the groove portion communicates with the second through hole, and the protrusion portion extends through the second through hole;
an end surface of the protrusion portion faces and is spaced apart from a bottom wall of the groove portion, and a peripheral side wall of the protrusion portion is slidably connected to a peripheral side wall of the groove portion; and
the protrusion portion is disposed on the first conductive member and the groove portion is defined on the second conductive member, or the protrusion portion is disposed on the second conductive member and the groove portion is defined on the first conductive member.

16. The electronic device of any one of claims 2-8 or 11-15, wherein
the housing frame defines a receiving groove on an outer peripheral wall of the housing frame, the first through hole communicates with the receiving groove and the inner cavity, and the first conductive member is at least partially received in the receiving groove; and
the electronic device further comprises a first elastic member and a second elastic member that are at least partially received in the receiving groove, the first elastic member and the second elastic member are arranged at opposite sides of the first through hole, respectively, and both the first elastic member and the second elastic member elastically abut against a bottom of the receiving groove and the first conductive member.

17. The electronic device of claim 16, further comprising a first limiting member and a second limiting member received in the receiving groove, wherein the first limiting member and the second limiting member are configured to limit opposite ends of the first conductive member, respectively.

18. The electronic device of claim 17, wherein
the first conductive member comprises a main body, a first hook portion, and a second hook portion, wherein both the first hook portion and the second hook portion are arranged on a side surface of the main body facing the bottom of the receiving groove;
the first conductive member moves toward the bottom of the receiving groove when being pressed by an external pressure; and
when the external pressure exerted on the first conductive member is removed, the first conductive member moves away from the bottom of the receiving groove until the first hook portion abuts against the first limiting member and the second hook portion abuts against the second limiting member.

19. The electronic device of any one of claims 1-8, 11-15, 17, or 18, further comprising a sealing ring, wherein the sealing ring is sleeved on a peripheral side wall of the second conductive member, and is sealingly connected between the peripheral side wall of the second conductive member and a hole wall of the first through hole.

20. The electronic device of any one of claims 1-8, 11-15, 17, or 18, wherein the circuit assembly further comprises a signal acquisition circuit disposed on the circuit board, the electrical contact portion is connected to the signal acquisition circuit, and the first conductive member is configured to serve as a detection electrode for detecting biological information.

21. The electronic device of claim 20, further comprising a bottom housing covering a side surface of the housing frame and provided with a third conductive member, wherein the third conductive member is at least partially disposed on an outer surface of the bottom housing, the third conductive member is connected to the signal acquisition circuit, and the third conductive member and the first conductive member cooperatively serve as the detection electrode for detecting biological information.

22. The electronic device of claim 21, wherein
the circuit assembly further comprises a functional circuit and a switch unit connected to the switch unit;
the electrical contact portion is a conductive resilient piece and comprises a fixed end and an abutting end connected to the fixed end, wherein the fixed end is fixed to the circuit board, and the fixed end is connected to the signal acquisition circuit, and wherein the abutting end is opposite to and spaced apart from the switch unit, and abuts the end of the second conductive member away from the first conductive member; and
when the first conductive member is pressed to push the second conductive member to move, the second conductive member pushes the abutting end to press against the switch unit, so that the functional circuit is triggered to generate a trigger signal.

23. A wearable electronic device, comprising a wearable member and the electronic device of any one of claims 1-22, wherein the wearable member is connected to the electronic device, and the electronic device comprises at least one detection electrode, and wherein the at least one detection electrode contacts a detection site of an object to-be-detected when the wearable electronic device is worn on the object to-be-detected.
